(19) Europäisches Patentamt
European Patent Office
Office européen des brevets

(11) Veröffentlichungsnummer: **0 299 028 B1**

# (12) EUROPÄISCHE PATENTSCHRIFT

(45) Veröffentlichungstag der Patentschrift: **16.12.92**

(51) Int. Cl.[5]: **C12P 13/04**, //(C12P13/04, C12R1:365)

(21) Anmeldenummer: **88901022.9**

(22) Anmeldetag: **22.01.88**

(86) Internationale Anmeldenummer: **PCT/DE88/00037**

(87) Internationale Veröffentlichungsnummer: **WO 88/05468 (28.07.88 88/17)**

## (54) VERFAHREN ZUR HERSTELLUNG VON L-AMINOSÄUREN.

(30) Priorität: **23.01.87 DE 3702384**

(43) Veröffentlichungstag der Anmeldung: **18.01.89 Patentblatt 89/03**

(45) Bekanntmachung des Hinweises auf die Patenterteilung: **16.12.92 Patentblatt 92/51**

(84) Benannte Vertragsstaaten: **AT BE CH DE FR GB IT LI LU NL SE**

(56) Entgegenhaltungen:

**GB-A- 204 531**

**Chemical Abstracts, vol. 93, No. 5, 4 August 1980, (Columbus, Ohio, US), see page 713, abstract 43950x, & JP, A, 8023994 (AJINOMOTO CO., INC.) 20 February 1980**

**Chemical Abstracts, vol. 107, No. 1, July 1987, (Columbus, Ohio, US), see pages 538-539, Abstract 5729g, & JP, A, 6200271 (DAIICHI KAGAKU YAKUHIN K.K.) 6 January 1987**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **KLAGES, Uwe**
**Schramberger Str. 19**
**W-1000 Berlin 28(DE)**
Erfinder: **WEBER, Alfred**
**Schützallee 56**
**W-1000 Berlin 37(DE)**
Erfinder: **WILSCHOWITZ, Ludwig**
**Mageritenstr. 7**
**W-8902 Neusaess(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft ein Verfahren zur Herstellung von L-Aminosäuren der allgemeinen Formel I

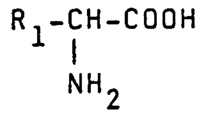

(I),

worin
$R_1$ einen gegebenenfalls durch Hydroxygruppen, Mercaptogruppen, Halogenatome, Aminogruppen, Carbonylgruppen oder Guanidinogruppen substituierter und/oder durch Sauerstoffatome, Stickstoffatome oder Schwefelatome unterbrochener Alkylrest mit maximal 12 Kohlenstoffatomen bedeutet, und im Falle der Mercaptoverbindungen der Formel I auch deren Dithioverbindungen, welches dadurch gekennzeichnet ist, daß man den Mikroorganismus Nocardia spec. DSM 3306 auf ein D,L-Imidazolidindion-Derivat der allgemeinen Formel II

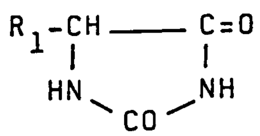

(II),

worin
$R_1$ die obengenannte Bedeutung besitzt oder im Falle der Mercaptoverbindungen der Formel II auch auf deren Dithioverbindungen einwirken läßt.

Die D,L-Imidazolidindion-Derivate der allgemeinen Formel II und demzufolge auch die daraus hergestellten L-Aminosäuren der allgemeinen Formel I können als Substituenten $R_1$ beispielsweise die Methylgruppe, die Ethylgruppe, die Propylgruppe, die 1-Methylethylgruppe, die Butylgruppe, die 1-Methylpropylgruppe, die 2-Methylpropylgruppe, die 1,1-Dimethylethylgruppe, die Pentylgruppe, die 1-Methylbutylgruppe, die 3-Methylbutylgruppe oder die Hexylgruppe tragen. Besonders bevorzugte Alkylgruppen $R_1$ sind solche mit maximal 6 Kohlenstoffatomen, wie die Methylgruppe des Verfahrensprodukts Alanin, die 1-Methylethylgruppe des Valins, die 2-Methylpropylgruppe des leucins, die 1-Methylpropylgruppe des Isoleucins oder die Ethylgruppe der $\alpha$-Aminobuttersäure.

Die Alkylgruppen $R_1$ können gegebenenfalls durch Hydroxygruppen, Mercaptogruppen, Halogenatome, Aminogruppen, Carbonylgruppen oder Guanidinogruppen substituiert sein, wobei einfach substituierte Alkylgruppen bevorzugt sind, oder durch Sauerstoffatome (vorzugsweise eins), Stickstoffatome (vorzugsweise eins oder zwei) oder Schwefelatome (vorzugsweise eins) unterbrochen sein. Als Alkylgruppen, die durch Hydroxygruppen oder Mercaptogruppen substituiert sind, seien besonders hervorgehoben die Hydroxymethylgruppe des Serins, die 1-Hydroxyethylgruppe des Threonins, die Mercaptomethylgruppe des Cysteins, die 2-Mercaptoethylgruppe des Homocysteins und die 1-Mercapto-1-methyl-ethylgruppe des $\beta$-Thiovalins. Als eine durch ein Schwefelatom unterbrochene Alkylgruppe $R_1$ sei die 2-Methylthioethylgruppe des Methionins hervorgehoben. Alkylgruppen $R_1$, die als Substituenten $R_1$ eine Aminogruppe oder eine Guanidinogruppe tragen sind beispielsweise die 2-Aminoethylgruppe der $\alpha,\gamma$-Diaminobuttersäure, die 3-Aminopropylgruppe des Ornithins, die 3-Guanidinopropylgruppe des Arginins und die 4-Aminobutylgruppe des lysins. Als Oxoalkylgruppen $R_1$ kommen vorzugsweise solche in Betracht, die zusätzlich noch durch eine Hydroxygruppe, Aminogruppe oder Guanidinogruppe substituiert und/oder durch ein Sauerstoffatom oder ein Stickstoffatom - genauer eine Iminogruppe - unterbrochen sind; derartige Gruppen sind beispielsweise die Acetoxymethylgruppe des O-Acetylserins, die 1-Acetoxyethylgruppe des 0-Acetylthreonins, die Carboxymethylgruppe der Asparaginsäure, die 2-Carboxyethylgruppe der Glutaminsäure, die 2-Methoxy-2-oxoethylgruppe des $\omega$-Asparaginsäuremonomethylesters, die 3-Ureidopropylgruppe des Citrulins oder die 3-Guanidino-3-oxo-propylgruppe des Canavanins.

Das erfindungsgemäße Verfahren wird unter Verwendung des Mikroorganismus Nocardia spec. DSM 3306 durchgeführt. Dieser Mikroorganismus wurde aus Erdproben isoliert, indem man diese mit einem Mineralsalzmedium, welches 5-(2-Methylpropyl)-hydantoin als einzige Stickstoffquelle enthielt, versetzte, incubierte und die gewachsenen Kulturen auf Agarplatten, die ebenfalls 5-(2-Methylpropyl)-hydantoin als einzige Stickstoffquelle enthielten, ausplattete.

Der erhaltene Mikroorganismus wurde bei der Deutschen Sammlung für Mikroorganismen hinterlegt und erhielt dort die Nummer DSM 3306. Er steht der Fachwelt unwiderruflich zur Verfügung.

Er besitzt taxonomisch folgende Eigenschaften:

| | |
|---|---|
| Koloniemorphologie | rund, unregelmäßiger Rand, nicht durchscheinend, |
| Zellmorphologie | in jungen Kulturen Stäbchen 1,2 µm dick, 8-20 µm lang, fragmentieren zu Stäbchen von 2-5 µm Länge, unverzweigt, unbeweglich |
| Gram-Färbung | gram-positiv |
| Säurefestigkeit | negativ |
| Endosporen | negativ |
| Sauerstoffverhältnis | obligat aerob |
| Katalase | positiv |
| Oxidase | positiv |
| Temperaturoptimum | 30-37° C |
| Citratverwertung | negativ |
| Nitrit aus Nitrat | positiv |
| Indolbildung | negativ |
| Methylrot | negativ |
| Voges-Proskauer | negativ |
| Urease | negativ |
| $H_2S$-Bildung | negativ |
| Gelatineverflüssigung | positiv |
| Stärkehydrolyse | positiv |
| Zuckerverwertung | Säure aus Saccharose, Glucose, Fructose, Arabinose keine Gasbildung |
| NaCl-Toleranz | bis 5%. |

Aufgrund seiner morphologischen und physiologischen Eigenschaften wurde der Stamm nach "Bergey's Manual of Determinative Bacteriology", 8. Auflage (1974), vorläufig der Gattung Nocardia zugeordnet.

Dieser Mikroorganismus wird unter den üblicherweise verwendeten Kulturbedingungen in einem geeigneten Nährmedium unter Belüften, Submerskulturen angezüchtet. Dann setzt man der Kultur das Substrat (vorzugsweise in einem geeigneten Lösungsmittel gelöst) zu und fermentiert, bis eine maximale Substratumwandlung erreicht ist.

Geeignete Substratlösungsmittel sind beispielsweise Wasser, Methanol, Äthanol, Glykolmonomethyläther, Dimethylformamid oder Dimethylsulfoxyd.

Die optimale Substratkonzentration, Substratzugabezeit und Fermentationsdauer ist von der Struktur des verwendeten Substrates und der Art der verwendeten Fermentationsbedingungen abhängig. Diese Größen müssen, wie dies bei mikrobiologischen Steroidumwandlungen allgemein erforderlich ist, im Einzelfall durch Vorversuche, wie sie dem Fachmann geläufig sind, ermittelt werden. Während der Fermentation wird der pH-Wert der fermentationsbrühe vorzugsweise auf einen pH-Wert von 7,5-10 eingestellt.

Andererseits ist es aber auch möglich, den angezüchteten Mikroorganismus beispielsweise durch Filtration oder Zentrifugieren vom Kulturmedium abzutrennen, ihn gewünschtenfalls mittels einer der bekannten Methoden zu immobilisieren und die Fermentation der Substrate mit der isolierten Zellmasse im resting cell Verfahren oder mittels der Immobilisate durchzuführen.

Die nachfolgenden Ausführungsbeispiele dienen zur Erläuterung des erfindungsgemäßen Verfahrens.

Beispiel 1

Ein 500 ml Erlenmeyerkolben mit 100 ml sterilem Nährmedium enthaltend 0,5 g Fleischextrakt, 0,5 g Pepton, 0,5 g Hefeextrakt und 0,2 g Natriumchlorid wird mit Nocardia spec. DSM 3306 beimpft und 20 Stunden lang bei 30° C mit 180 Umdrehungen/Minute geschüttelt. Dann trennt man die Zellmasse durch Zentrifugieren ab und wäscht sie mit physiologischer Kochsalzlösung.

400 mg der feuchten Zellmasse werden in 10 ml 0,1 M Tris/HCl-Puffer vom pH 8,5 suspendiert, mit 10 mg 5-(2-Methylpropyl)-hydantoin versetzt und 24 Stunden lang bei 30° C incubiert. Durch Bestimmung mit L-Aminosäure-Oxidase wird ermittelt, daß 7,9 mg L-Leucin gebildet werden.

Beispiel 2

Unter den Bedingungen des Beispiels 1 werden aus 10 mg 5-(1-Methylpropyl)-hydantoin 3,5 mg L-Isoleucin gebildet.

Beispiel 3

Unter den Bedingungen des Beispiels 1 werden aus 10 mg 5-(1-Methylethyl)-hydantoin 2,5 mg L-Valin gebildet.

Beispiel 4

Unter den Bedingungen des Beispiels 1 werden aus 10 mg 5,5'-Dithiobismethylen-bis-hydantoin 2,1 mg Cystin gebildet.

Beispiel 5

Ein 2 l Erlenmeyerkolben mit 500 ml sterilem Nährmedium enthaltend 2,5 g Fleischextrakt, 2,5 g Pepton, 2,5 g Hefeextrakt und 1 g Natriumchlorid wird mit 50 ml einer 20 Stunden alten Kultur von Nocardia spec. DSM 3306 - hergestellt nach Beispiel 1 - beimpft und 20 Stunden lang bei 30° C mit 180 Umdrehungen pro Minute geschüttelt. Die Zellmasse wird abzentrifugiert und mit physiologischer Kochsalzlösung gewaschen.

8 g der feuchten Zellmasse werden in 200 ml 0,1 M Tris/HCl-Puffer vom pH 8,5 suspendiert mit 1,0 g 5-(2-Methylpropyl)-hydantoin versetzt und 24 Stunden lang bei 30° C incubiert. Man zentrifugiert die Zellmasse dann ab und isoliert aus dem Filtrat 660 mg L-Leucin vom Zersetzungspunkt 291° C (aus wäßrigem Ethanol) $[\alpha]_D^{20}$ = + 15,6° (20 %ige wäßrige Salzsäure).

Beispiel 6

a) 3,5 g feuchte Zellmasse von Nocardia spec. DSM 3306 - hergestellt nach Beispiel 5 - werden in 31,5 g einer 2 %igen wässrigen Lösung von Natriumalginat suspendiert und in 500 ml einer 0,1 M wässerigen lösung von Calziumchlorid-Dihydrat getropft.

b) 1,5 g des so erhaltenen Immobilisats werden in 10 ml 0,1 M Tris/HCl-Puffer vom pH 8,5 suspendiert mit 10 mg 5(2-Methylpropyl)-hydantoin versetzt und 18 Stunden lang bei 30° C incubiert. Durch Bestimmung mit L-Aminosäure-Oxidase wird ermittelt, daß 7,8 mg L-Leucin gebildet werden.

Beispiel 7

Unter den Bedingungen des Beispiels 6 b werden aus 10 mg 5-(1-Methylpropyl)-hydantoin 3,2 mg L-Isoleucin gebildet.

Beispiel 8

Unter den Bedingungen des Beispiels 6 b werden aus 10 mg 5-(1-Methyl-ethyl)-hydantoin 2,1 mg L-Valin gebildet.

Beispiel 9

Unter den Bedingungen des Beispiels 6 b werden aus 10 mg 5,5'-Dithiobismethylen-bis-hydantoin 4,1 mg Cystin gebildet.

**Patentansprüche**

**1.** Verfahren zur Herstellung von L-Aminosäuren der allgemeinen Formel I

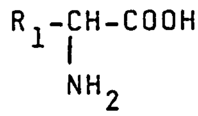 (I),

worin
$R_1$ einen gegebenenfalls durch Hydroxygruppen, Mercaptogruppen, Halogenatome, Aminogruppen, Carbonylgruppen oder Guanidinogruppen substituierter und/oder durch Sauerstoffatome, Stickstoffatome oder Schwefelatome unterbrochener Alkylrest mit maximal 12 Kohlenstoffatomen bedeutet, und im Falle der Mercaptoverbindungen der Formel I auch deren Dithioverbindungen,
dadurch gekennzeichnet, daß man den Mikroorganismus Nocardia spec. DSM 3306 auf ein D,L-Imidazolidindion-Derivat der allgemeinen Formel II

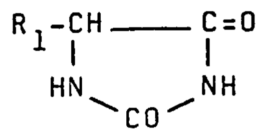 (II),

worin
$R_1$ die obengenannte Bedeutung besitzt oder im Falle der Mercaptoverbindungen der Formel II auch auf deren Dithioverbindungen einwirken läßt.

**2.** Verfahren zur Herstellung von L-Aminosäuren der allgemeinen Formel I a

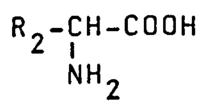 (Ia),

worin
$R_2$ einen gegebenenfalls durch eine Hydroxygruppe, eine Mercaptogruppe, eine Methylthiogruppe, eine Aminogruppe, eine Carboxylgruppe oder Guanidinogruppe substituierter Alkylrest mit maximal 6 Kohlenstoffatomen bedeutet und im Falle der Mercaptoverbindungen der Formel I a auch deren Dithioverbindungen, dadurch gekennzeichnet, daß man den Mikroorganismus Nocardia spec. DSM 3306 auf ein D,L-Imidazolidindion-Derivat der allgemeinen Formel II a

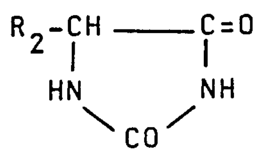 (IIa),

worin
$R_2$ die obengenannte Bedeutung besitzt oder im Falle der Mercaptoverbindungen der Formel II auch auf deren Dithioverbindungen einwirken läßt.

**3.** Verfahren zur Herstellung von L-Aminosäuren der allgemeinen Formel I b

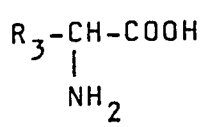 (Ib),

worin

$R_3$ eine Alkylgruppe mit maximal 6 Kohlenstoffatomen, eine Mercaptomethylgruppe oder die Gruppierung der Formel III

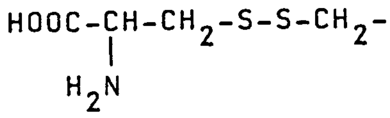

(III)

bedeutet,
dadurch gekennzeichnet, daß man den Mikroorganismus Nocardia spec. DSM 3306 auf ein D,L-Imidazolidindion-Derivat der allgemeinen Formel II b

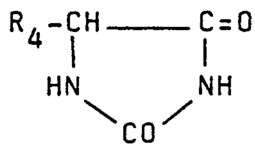

(IIb),

worin
$R_4$ eine Alkylgruppe mit maximal 6 Kohlenstoffatomen, eine Mercaptomethylgruppe oder die Gruppierung der Formel IV

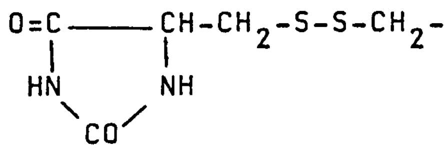

(IV),

bedeutet, einwirken läßt.

## Claims

**1.** Process for the preparation of L-amino acids of the general formula I

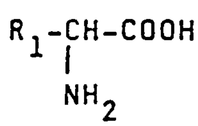

(I)

wherein
$R_1$ represents an alkyl radical having a maximum of 12 carbon atoms that is optionally substituted by hydroxy groups, mercapto groups, halogen atoms, amino groups, carbonyl groups or guanidino groups and/or optionally interrupted by oxygen atoms, nitrogen atoms or sulphur atoms, and, in the case of the mercapto compounds of the formula I, also the dithio compounds thereof, characterised in that the micro-organism Nocardia spec. DSM 3306 is allowed to act on a D,L-imidazolidinedione derivative of the general formula II

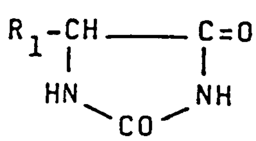

(II)

wherein

$R_1$ is as defined above or, in the case of the mercapto compounds of the formula II, alternatively on the dithio compounds thereof.

**2.** Process for the preparation of L-amino acids of the general formula Ia

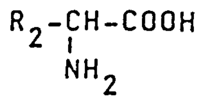

(Ia)

wherein
$R_2$ represents an alkyl radical having a maximum of 6 carbon atoms that is optionally substituted by a hydroxy group, a mercapto group, a methylthio group, an amino group, a carboxyl group or a guanidino group, and, in the case of the mercapto compounds of the formula Ia, also the dithio compounds thereof, characterised in that the micro-organism Nocardia spec. DSM 3306 is allowed to act on a D,L-imidazolidinedione derivative of the general formula IIa

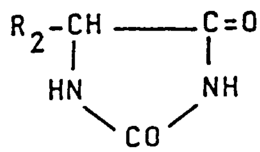

(IIa)

wherein
$R_2$ is as defined above or, in the case of the mercapto compounds of the formula II, alternatively on the dithio compounds thereof.

**3.** A process for the preparation of L-amino acids of the general formula Ib

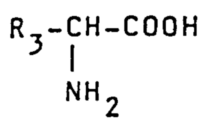

(Ib)

wherein
$R_3$ represents an alkyl group having a maximum of 6 carbon atoms, a mercaptomethyl group or the grouping of the formula III

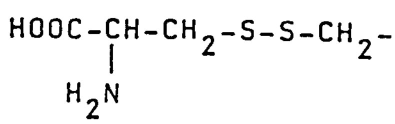

(III)

characterised in that the micro-organism Nocardia spec. DSM 3306 is allowed to act on a D,L-imidazolidinedione derivative of the general formula IIb

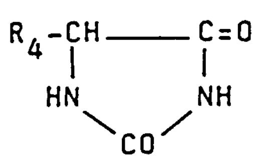

(IIb)

wherein

$R_4$ represents an alkyl group having a maximum of 6 carbon atoms, a mercaptomethyl group or the grouping of the formula IV

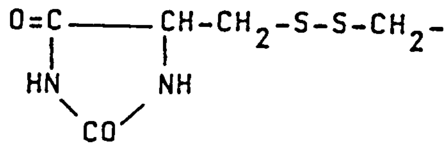 (IV).

## Revendications

**1.** Procédé pour préparer des amino-acides L répondant à la formule générale I :

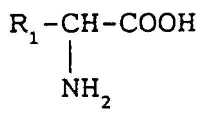 (I)

dans laquelle $R_1$ représente un radical alkyle qui contient au plus 12 atomes de carbone, porte éventuellement des radicaux hydroxy, des radicaux mercapto, des atomes d'halogène, des radicaux amino, des radicaux oxo ou des radicaux guanidino et/ou est éventuellement interrompu par des atomes d'oxygène, d'azote ou de soufre,
et, dans le cas des composés mercapto de formule I, également leurs dérivés dithio, procédé caractérisé en ce qu'on fait agir le micro-organisme Nocardia spec. DSM 3306 sur une imidazolidine-dione D,L répondant à la formule générale II :

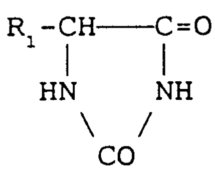 (II)

dans laquelle $R_1$ a la signification indiquée ci-dessus, ou, dans le cas de composés mercapto de formule II, également sur leurs dérivés dithio.

**2.** Procédé pour préparer des amino-acides L répondant à la formule générale Ia :

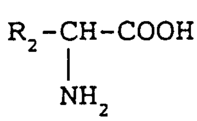 (Ia)

dans laquelle $R_2$ représente un radical alkyle qui contient au plus 6 atomes de carbone et qui porte éventuellement un radical hydroxy, un radical mercapto, un radical méthylthio, un radical amino, un radical carboxy ou un radical guanidino,
et, dans le cas des composés mercapto de formule Ia, également leurs dérivés dithio, procédé caractérisé en ce qu'on fait agir le micro-organisme Nocardia spec. DSM 3306 sur une imidazolidine-dione D,L répondant à la formule générale IIa :

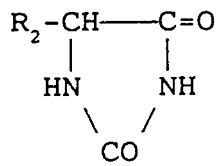

(IIa)

dans laquelle $R_2$ a la signification indiquée ci-dessus, ou, dans le cas de composés mercapto de formule IIa, également sur leurs dérivés dithio.

**3.** Procédé pour préparer des amino-acides L répondant à la formule générale Ib :

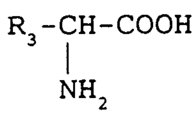

(Ib)

dans laquelle $R_3$ représente un radical alkyle qui contient au plus 6 atomes de carbone, un radical mercaptométhyle ou le radical de formule III :

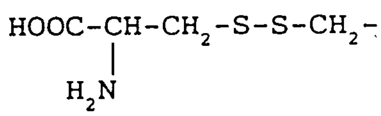

(III),

procédé caractérisé en ce qu'on fait agir le microorganisme Nocardia spec. DSM 3306 sur une imidazolidinedione D,L répondant à la formule générale IIb :

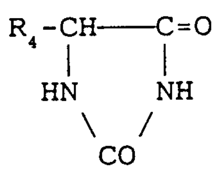

(IIb)

dans laquelle $R_4$ représente un radical alkyle contenant au plus 6 atomes de carbone, un radical mercaptométhyle ou un radical de formule IV :

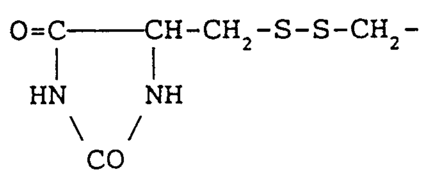

(IV).